# EUROPEAN PATENT SPECIFICATION

(11) **EP 2 677 868 B1**
(45) Date of publication and mention of the grant of the patent: **15.01.2020**
(21) Application number: 12749531.5
(22) Date of filing: 17.02.2012
(51) Int. Cl.: A61K 9/20, A61K 31/46, A61K 9/16, A61P 31/18

(54) **PHARMACEUTICAL COMPOSITIONS OF MARAVIROC AND PROCESS FOR THE PREPARATION THEREOF**
PHARMAZEUTISCHE ZUSAMMENSETZUNGEN AUS MARAVIROC UND VERFAHREN ZU IHRER HERSTELLUNG
COMPOSITIONS PHARMACEUTIQUES DE MARAVIROC ET LEUR PROCÉDÉ DE PRÉPARATION

(30) Priority: 21.02.2011 IN 491CH2011
(43) Date of publication of application: 01.01.2014
(73) Proprietor: Hetero Research Foundation, Telangana (IN)
(72) Inventor: PARTHASARADHI REDDY, Bandi, Hyderabad 500 018 Telangana (IN); KHADGAPATHI, Podili, Hyderabad 500 018 Telangana (IN); KAMALAKAR REDDY, Goli, Hyderabad 500 018 Telangana (IN); RAMA RAO, nelluri, Hyderabad 500 018 Telangana (IN)
(74) Representative: ZBM Patents ApS
(86) International application number: PCT/IN2012/000115
(87) International publication number: WO 2012/114352

(56) References cited:
- WO-A1-2010/048593
- WO-A1-2010/143207
- WO-A2-2009/002823
- US-A1- 2007 197 478
- US-A1- 2010 239 667
- US-A1- 2010 267 765
- US-A1- 2011 039 803
- US-A1- 2011 123 555
- US-B2- 6 667 314
- "SCIENTIFIC DISCUSSION", EMEA 2007, 2007, pages 1-52, XP055123672, Retrieved from the Internet: URL:http://www.ema.europa.eu/docs/en_GB/do cument_library/EPAR_-_Scientific_Discussio n/human/000811/WC500022194.pdf [retrieved on 2014-06-17]

## Description

This patent application claims priority to Indian application number 491/CHE/2011, filed on February 21, 2011.

### Field of the Disclosure

The present disclosure relates to solid dosage forms comprising maraviroc, a CCR5 co-receptor antagonist. More particularly, the present disclosure relates to solid oral dosage forms containing maraviroc, and process for preparing the same.

### Background of the Disclosure

Maraviroc is chemically, N-{(1S)-3-[3-(3-isopropyl-5-methyl-4H-1,2,4-triazol-4-yl)-exo-8-azabicyclo-[3.2.1]oct-8-yl]-1-phenylpropyl}-4,4 difluoro cyclohexane carboxamide, having the following structural formula:

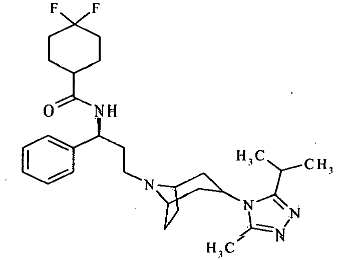

Maraviroc is a modulator of the chemokine receptor CCR5 and is useful in the treatment of retroviral diseases caused by viruses that utilize CCR5 to enter cells. In particular, maraviroc has been disclosed as a useful therapeutic agent in the treatment of HIV, a retroviral infection genetically related to HIV AIDS, and also inflammatory disease.

Maraviroc is marketed under the trade name Selzentry® in United States by ViiV Healthcare in the form of oral tablet.

Maraviroc and its pharmaceutically acceptable salts and solvates thereof are disclosed in U.S. Patent No. 6,667,314. Said patent also describes solid dosage forms like tablets comprising the active compound and excipients.

U.S. Patent No. 7,576,097 assigned to Pfizer discloses crystalline Form A and Form B of maraviroc.

What is needed are improved pharmaceutical compositions containing maraviroc as an active agent.

### Summary

Described herein are solid oral compositions comprising maraviroc and a pharmaceutically acceptable excipient, prepared either by direct compression or dry granulation process.

In one aspect, described herein is a solid oral composition comprising maraviroc, a diluent, a disintegrant and colloidal silicon dioxide as a dispersing agent, wherein the solid oral composition is prepared either by direct compression or dry granulation by slugging.

Another aspect provides disintegration time of the composition is less than 2 minutes when measured by a USP disintegration apparatus at 37°C ± 2°C.

In another aspect, a solid oral composition in the form of a tablet comprises i) 20 wt% to 30 wt% of amorphous maraviroc having a particle size d₉₀ not more than 150µm; ii) 0.5 wt% to 5 wt% of the colloidal silicon dioxide as a dispersing agent; iii) 40 wt% to 80 wt% of the diluent; and iv) 1 wt% to 8 wt% of the disintegrant based on the total weight of the composition.

In yet another aspect, a tablet composition comprises maraviroc, microcrystalline cellulose, lactose, colloidal silicon dioxide, magnesium stearate and one or more disintegrants selected from croscarmellose sodium, sodium starch glycolate, crospovidone and polacrilin potassium; where in the tablet is prepared by either direct compression or dry granulation by slugging.

In a further aspect, a process for the preparation of tablet composition comprising maraviroc and a pharmaceutically acceptable excipient comprises: (i) dry mixing maraviroc with a diluent, a disintegrant and colloidal silicon dioxide, (ii) blending the dry mix to form a blend, (iii) lubricating the blend and finally (iv) compressing the lubricated blend of step (iii) into tablets.

In another aspect, a process for preparing a compressed tablet composition comprising maraviroc comprises (a) sifting and blending the maraviroc with a diluent, a disintegrant and colloidal silicon dioxide to form a blended mixture, (b) compressing the blended mixture of step (a) to form slugs, (c) sizing the slugs to form granules; (d) blending the granules with an excipient selected from a binder, a lubricant and a glidant; and (e) compressing the granules of step (d) in to tablets.

In another aspect, included herein is a solid oral composition comprising amorphous maraviroc having a particle size d₉₀ not more than 150µm, preferably 5- 100µm, more preferably 10-80µm.

In yet another aspect, included herein is a method of improving patient compliance in a patient in need of treatment of HIV-1 comprising administering the solid oral dosage forms disclosed herein.

### Detailed Description

Described herein are novel maraviroc solid oral dosage forms that have favorable disintegration and dissolution characteristics. The inventors of the present application have found that by using a combination of a disintegrant and a particular dispersing agent, tablets having favorable disintegration times and dissolution characteristics can be produced, thereby providing bioequivalent compositions. Further, inventors of the present invention have surprisingly found that amorphous maraviroc having a particle size d₉₀ less than 150µm was found to exhibit excellent in-vitro and in-vivo characteristics that were also found to be comparable with the marketed formulation.

As used herein, term "maraviroc" includes maraviroc in the form of a free base or its pharmaceutically acceptable salts, amorphous maraviroc, crystalline maraviroc and any isomers, hydrate and solvates thereof.

Disclosed herein are solid oral compositions comprising maraviroc, a diluent, a disintegrant and a colloidal silicon dioxide as dispersing agent. The compositions optionally further comprise binders, lubricants and glidants.

Solid oral compositions include tablets, capsules, and granules.

A "pharmaceutical composition" comprises an active pharmaceutical ingredient and a pharmaceutically acceptable excipient. The term "pharmaceutically acceptable excipient" includes a pharmaceutically acceptable material, composition or vehicle, suitable for administering an active pharmaceutical ingredient. Each excipient should be "acceptable" in the sense of being compatible with the other ingredients of the formulation and not injurious to the patient. Excipients include diluents, binders, disintegrants, glidants, lubricants and others.

Exemplary diluents (also called fillers) include lactose, sugar, starches, modified starches, mannitol, sorbitol, inorganic salts, cellulose derivatives (e.g., microcrystalline cellulose), calcium sulfate, xylitol, lactitol, starch, pregelatinized starch, kaolin, sucrose, mannitol, sorbitol, dextrates, dextrin, maltodextrin, dextrose, calcium carbonate, calcium sulfate, dibasic calcium phosphate dihydrate, tribasic calcium phosphate, magnesium carbonate, magnesium oxide, and the like, and mixtures thereof.

The term "disintegrant" as used herein means a compound used in solid dosage forms to promote the disruption of the solid mass into smaller particles which are more readily dispersed or dissolved. Suitable disintegrants include, by way of example and without limitation, polacrillin potassium, croscarmellose sodium, crospovidone (e.g., KOLLIDON®, POLYPLASDONE®), polyvinylpyrrolidone, sodium starch glycolate (e.g., PRIMOGEL, EXPLOTAB®), hydroxypropylmethylcellulose, hydroxypropyl cellulose, carboxymethylcellulose calcium, starches such as corn starch, potato starch, pre-gelatinized starch and modified starches, clays, bentonite, microcrystalline cellulose (e.g., Avicel™), carsium (e.g., Amberlite™), alginates, gums such as agar, guar, locust bean, karaya, pectin, tragacanth, and the like, and combinations thereof.

The term "dispersing agent" as used herein means a substance used to promote the disintegration and/ or dissolution of tablet. Suitable dispersing agents include colloidal silicon dioxide, calcium silicate, magnesium trisilicate, silicon hydrogel and silica derivatives. A preferred dispersing agent as per the present invention is colloidal silicon dioxide.

The term "binders" as used herein means substances used to cause adhesion of powder particles in tablet granulations. Suitable binders include, by way of example and without limitation, lactose, starches such as corn starch, potato starch, modified starches, sugars, guar gum, pectin, wax binders, microcrystalline cellulose, methylcellulose, carboxymethylcellulose, hydroxypropylmethylcellulose, hydroxyethyl cellulose, hydroxypropyl cellulose, copolyvidone, sodium alginate, acacia, alginic acid, tragacanth, carboxymethylcellulose sodium, ethyl cellulose, gelatin, liquid glucose, povidone, pregelatinized starch, and the like, and mixtures thereof.

The term "lubricant" as used herein means substances used to reduce friction during tablet compression. Suitable lubricants include, by way of example and without limitation, calcium stearate, magnesium stearate, zinc stearate, mineral oil, stearic acid, fumaric acid, palmitic acid, talc, carnauba wax, hydrogenated vegetable oils, mineral oil, polyethylene glycols, sodium stearyl fumarate, and the like, and combinations thereof.

The term "glidant" as used herein means agents used in tablet and capsule formulations to improve flow-properties during tablet compression and to produce an anti-caking effect. Such compounds include, by way of example and without limitation, colloidal silica, calcium silicate, magnesium silicate, silicon hydrogel, cornstarch, talc, and the like, and combinations thereof.

The solid oral dosage forms, e.g., tablets, disclosed herein are optionally coated with an aqueous or non aqueous solution or dispersion of film forming agents. In one embodiment, the film coat is an aqueous moisture barrier. The coating solution comprises film forming polymers and one or more of plasticizers, opacifier, surfactant, anti-tacking agents, coloring agents and the like, and combinations thereof.

The coating is applied by solubilising or suspending the excipients in solvents such as isopropyl alcohol, water, acetone, ethanol, methylene chloride, and the like, and mixtures thereof.

In one embodiment, a composition comprises 22-27 wt% of maraviroc, 50-75 wt% of a diluent, 1-8 wt% of a disintegrant, optionally 0.5-2 wt% of a lubricant, optionally 0.1-4 wt% of a glidant and optionally 1-4 wt% of a coating material based on the total weight of the tablet, wherein the tablet is prepared by either direct compression or dry granulation by slugging.

In one embodiment, the ratio of disintegrant to dispersing agent is 1:0.25 to 1:1.

In one embodiment, the total weight of the tablet is over 1000 mg. In a specific embodiment, the tablet comprises 300 mg of maraviroc.

In one embodiment, the disintegration time of the oral dosage form is less than 2 minutes, specifically less than 1 minute when measured at 37°C ± 2°C by USP disintegration apparatus.

Solid oral compositions are prepared by direct compression or dry granulation.

A direct compression process for preparing maraviroc tablets comprises (i) dry mixing and blending maraviroc with a diluent, a disintegrant and colloidal silicon dioxide, (ii) lubricating the blend obtained in step (i) by adding a lubricant and finally (iii) compressing the lubricated blend of step (ii) into tablets or filling the lubricated blend into capsules.

In one embodiment, dry granulation comprises (i) sifting and blending maraviroc with a diluent, disintegrant and colloidal silicon dioxide; (ii) compressing the blended mixture of step (i) to form slugs; (iii) sizing the slugs to form granules; (iv) blending the granules with an additional excipient to form blended granules; and (v) compressing the blended granules of step (iv) in to tablets or filling the blended granules into capsules. In one embodiment, the additional excipient comprises a binder, a lubricant or a glidant.

Alternatively, dry granulation comprises compacting maraviroc, a diluent, a disintegrant and colloidal silicon dioxide in a roller compactor, and passing the compacts through a sieve such as an ASTM sieve # 20 to obtain granules. The granules were lubricated and compressed into tablets on a rotary compression machine. The resulting tablets were optionally coated with Opadry.

In another embodiment, a tablet composition comprises, based on the total weight of the tablet, i) 20 wt% to 30 wt% of amorphous maraviroc having a particle size d₉₀ not more than 150µm; ii) 0.5 wt% to 5 wt% of a colloidal silicon dioxide dispersing agent; iii) 40 wt% to 80 wt% of a diluent; and iv) 1 wt% to 8 wt% of a disintegrant; where in the tablet is prepared either by direct compression or dry granulation by slugging.

Maraviroc as used herein may take any of the forms selected from amorphous maraviroc, crystalline Form A, Form B, Form 1, Form 2, Form 3 or Form 4 of maraviroc, or combinations thereof. In one embodiment, the maraviroc is amorphous maraviroc.

In one embodiment, a solid oral composition comprises amorphous maraviroc having a particle size d₉₀ not more than 150 µm, specifically 5- 100 µm, more specifically 10- 80 µm.

If the particle size of amorphous maraviroc is less than 10 µm, process issues like sticking, poor flow, weight variation may occur. If the particle size of amorphous maraviroc is more than 150 µm, the dissolution rate may be hindered which ultimately affects in-vivo performance of the drug product.

Particle size can be reduced and/or controlled using techniques such as milling. A desired particle size of maraviroc is obtained by a suitable micronization technique known in the art such dry milling, wet milling, air jet milling, sieving, homogenizing using homogenizer, such as rotor-stator and/or high pressure homogenizer such as a MICROFLUIDIZER and the like. Micronized maraviroc provides good *in vitro* end release and *in vivo* bioavailability.

An unformulated active pharmaceutical ingredient as disclosed herein can have a particle size defined by the particle size distribution. The D₁₀, or 10^{th} volume percentile, is the size of particles below which 10% of the measured particle volumes, lie; the D₅₀, or 50^{th} volume percentile, is the size of particles below which 50% of the measured particle volumes, lie; and the D₉₀, or 90^{th} volume percentile, is the size of particles below which 90% of the measured particle volumes, lie. Particle size distributions can be determined using laser diffraction using a Malvern Mastersizer, for example.

Pharmaceutical composition comprising a therapeutically effective amount of maraviroc as disclosed herein is useful for treating HIV-1 infections.

Also included herein are methods of improving patient compliance by administering the dosage forms with favorable disintegration times as disclosed herein.

The invention is further exemplified with following examples which are not intended to limit the scope of the invention.

### Examples

### Examples 1-3

**Maraviroc tablets compositions prepared by direct compression.**

| **S.No** | **Ingredients** | **Qty per unit** | | |
|---|---|---|---|---|
| | | **Example-1** | **Example-2** | **Comparative Example-3** |
| **Pre** - **mix** | | | | |
| 1 | Maraviroc amorphous | 300.000 | 300.000 | 300.000 |
| 2 | Microcrystalline cellulose | 709.125 | 640.625 | 663.625 |
| 3 | Lactose monohydrate | 57.500 | 126.000 | 126.000 |
| 4 | Colloidal silicon dioxide | 23.000 | 23.000 | --- |
| 5 | Sodium starch glycolate | 46.000 | 46.000 | 46.000 |

| **Final Mix** / **Lubrication** | | | | |
|---|---|---|---|---|
| 6 | Magnesium stearate | 14.375 | 14.375 | 14.375 |
| **Core Tablet Weight** | | 1150.000 | 1150.000 | 1150.000 |

| **Coating** (15% w/w Suspension) | | | | |
|---|---|---|---|---|
| 7 | Opadry II Blue 85G20583, | 20.125 | 20.125 | 20.125 |
| 8 | Purified water | 114.040 | 114.040 | 114.040 |
| **Total Tablet Weight** | | 1170.125 | 1170.125 | 1170.125 |
| **Disintegration time** | | Less than 1 min | Less than 1 min | 3 - 4 min |

The disintegration time was measured at 37°C ± 2°C by USP disintegration apparatus.

### Manufacturing process: .

i) Maraviroc, microcrystalline cellulose, lactose, colloidal silicon dioxide and sodium starch glycolate were sifted through mesh # 40.
ii) the materials of step no. (i) were loaded into a blender and mixed for 15 minutes.
iii) magnesium stearate was sifted through mesh #60 and added to the materials of step no. (ii) and blended for 5 minutes.
iv) the blend of step no. (iii) was compressed into tablets or filled into capsules.
v) the tablets of step no. (iv) were film coated using Opadry II Blue 85G20583.

### Dissolution data for Examples 1-3:

| **Dissolution in 900 ml of 0.01 N HCl, USP-I, 100 rpm** | | | | |
|---|---|---|---|---|
| **Time in min** | **Cumulative % Drug release** | | | |
| | **Selzentry® 300 mg (Innovator)** | **EX 1** | **EX 2** | **EX 3** |
| 5 | 62 | 56 | 66 | 38 |
| 10 | 80 | 77 | 85 | 62 |
| 15 | 88 | 89 | 94 | 76 |
| 20 | 92 | 95 | 96 | 87 |
| 30 | 95 | 98 | 96 | 93 |
| 45 | 97 | 98 | 97 | 96 |

Based on the dissolution data of Examples 1-3, it has been observed that, the maraviroc tablets of example 1 and 2 containing colloidal silicon dioxide exhibited an improved dissolution profile as compared to the pharmaceutical composition of comparative example 3.

### Example 4:

**Solid oral compositions of Maraviroc prepared by dry granulation by slugging:**

| **S. No** | **Ingredients** | **Mg / tablet** |
|---|---|---|
| **Intragranular** | | |
| 1 | Maraviroc | 300.00 |
| 2 | Microcrystalline cellulose | 500.00 |
| 3 | Corn starch | 126.00 |
| 4 | Croscarmellose sodium | 10.00 |
| 5 | Magnesium stearate | 6.00 |

| **Extragranular** | | |
|---|---|---|
| 6 | Microcrystalline cellulose | 190.00 |
| 7 | Colloidal silicon dioxide | 12.50 |
| 8 | Magnesium stearate | 5.50 |
| **Total tablet weight** | | 1150.00 |

### Manufacturing process:

i) Maraviroc, microcrystalline cellulose, corn starch, and croscarmellose sodium were sifted together through # 40 mesh.
ii) magnesium stearate sifted separately through # 60 mesh.
iii) material of step no .(i) & (ii) were mixed together for 10 minutes.
iv) the above material was slugged and the resulted slugs were milled using multimill or cone mill with 2mm screen.
v) milled granules of step (iv) were sifted through #30 mesh completely.
vi) microcrystalline cellulose, magnesium stearate and colloidal silicon dioxide of extra granular portion were sifted together through # 40 mesh, and added to the sifted granules of step (v) and blended for 10 minutes.
vii) the blend of step no. (vi) was compressed into tablets or filled into capsules.
viii) compressed tablets were optionally coated with Opadry II.

### Example 5:

A biostudy was conducted to compare the formulation of Example 2 with the commercially available Selzentry® 300 mg tablet. The studies were open-label, balanced, randomized, two-treatment, two-period, two-sequence cross-over, single-dose bioequivalence studies, in 50 healthy human subjects. The study was conducted under fed and fasting conditions. Selzentry® is described as a film-coated 300 mg tablet containing dibasic calcium phosphate, magnesium stearate, microcrystalline cellulose, and sodium starch glycolate. The coating is Opadry II Blue.

In certain embodiments, the formulations described herein are bioequivalent to the reference listed dosage form (RLD).

The study duration was 11 days with a washout period of 11 days within both treatment periods. The study parameters determined were Cₘₐₓ, Tₘₐₓ, AUC₀₋ₜ, AUC_{0-inf}, t_{1/2}, and Kₑₗ.

The pharmacokinetic parameters for the fed study are given below:

| **PK parameters** | **Maraviroc-test according to present disclosure** | **Maraviroc-commercial dosage form** |
|---|---|---|
| **Tₘₐₓ (hr)** | 3.68 ±1.4441 | 3.675±1.3087 |
| **Cₘₐₓ (ng/mL)** | 568.858±350.6957 | 547.161±384.8896 |
| **AUC₀₋ₜ (ng h/mL)** | 1934±1194.3276 | 1708.992±754.1851 |
| **AUC_{0-inf} (ng h/ML)** | 2255.734±1257.2403 | 1954.676±771.7304 |
| **T_{1/2} (hr)** | 5.653±4.3898 | 4.249±2.0422 |
| **Kₑₗ (1/hr)** | 0.198±0.1254 | 0.201±.0936 |

As can be seen from the table, the test maraviroc formulation according to the present disclosure is bioequivalent to the commercially available product under fed conditions.

The pharmacokinetic parameters for the fasting study are given below:

| **PK parameters** | **Maraviroc-test according to present disclosure** | **Maraviroc-commercial dosage form** |
|---|---|---|
| **Tₘₐₓ (hr)** | 2.981±1.1880 | 2.965±1.3131 |
| **Cₘₐₓ (ng/mL)** | 1035.898±381.7004 | 1121.380±402.6862 |
| **AUC₀₋ₜ (ng h/mL)** | 3573.856±1195.4966 | 3498.488±1115.9285 |
| **AUC_{0-inf} (ng h/ML)** | 3752.359±1215.8244 | 3679.678±1182.3191 |
| **T_{1/2} (hr)** | 2.850±0.0992 | 0.293±0.1019 |
| **Kₑₗ (1/hr)** | 4.993±3.0593 | 4.866±2.5526 |

As can be seen from the table, the test maraviroc formulation according to the present disclosure is bioequivalent to the commercially available product under fasting conditions.

As used herein, a polymorph is a crystalline form of an active pharmaceutical ingredient, and includes crystalline polymorphs, amorphous forms, as well as solvate and hydrate forms, which are often referred to as pseudopolymorphs. Solvates are crystalline forms containing a stoichiometric or nonstoichiometric amount of solvent. A hydrate is a solvate wherein the solvent is water.

Amorphous forms are disordered forms that do not have a distinguishable crystalline lattice. Amorphous materials typically do not have sharp, well-defined reflections in their x-ray diffraction patterns, but rather a broad peak spanning a range of two-theta angles.

An active pharmaceutical ingredient can be used as a pharmaceutically acceptable salt. As used herein a "pharmaceutically acceptable salt" is a salt of an acidic or basic group that is non-toxic. Exemplary acids used to form pharmaceutically acceptable salts include containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, mesylate, saccharate, formate, benzoate, glutamate, methanesulfonate, ethanesulfonate, benzenesulfonate, p-toluenesulfonate and pamoate salts. Exemplary bases include sodium hydroxide, sodium carbonate, potassium hydroxide, aluminum hydroxide, calcium hydroxide, ethanolamine, diethanolamine, triethanolamine, tromethamine, N-methylglucamine and the like.

The dissolution properties of a dosage form can be tested by methods known in the art. An exemplary condition is a USP type 2 (paddle) apparatus at 50 rpm in 900 ml of phosphate buffer with pH 7.5 and at 37°C. Alternatively, a basket method may be employed. An alternative set of conditions is 37°C for 2 hours in 0.1 M HCl, optionally followed by testing in a buffer pH 6.2.

All ranges disclosed herein are inclusive and combinable. While the invention has been described with reference to a preferred embodiment, it will be understood by those skilled in the art that it is intended that the invention not be limited to the particular embodiment disclosed as the best mode contemplated for carrying out this invention, but that the invention will include all embodiments falling within the scope of the appended claims.

## Claims

1. A solid oral composition comprising maraviroc, a diluent, a disintegrant, and colloidal silicon dioxide as a dispersing agent, wherein the solid oral composition is prepared either by direct compression or dry granulation.

2. The solid oral composition according to claim 1, selected from a tablet, a capsule and a granule.

3. The solid oral composition according to claim 1, further comprising a binder, a lubricant, a glidant, or a combination thereof.

4. The solid oral composition according to claim 1, wherein the diluent is selected from the group consisting of lactose, dibasic calcium phosphate, sucrose, glucose, mannitol, sorbitol, calcium carbonate, starch, microcrystalline cellulose, cellulose derivatives, and combinations thereof.

5. The solid oral composition according to claim 1, wherein the disintegrant is selected from croscarmellose sodium, crospovidone, polacrilin potassium, calcium silicate, carboxymethylcellulose sodium, sodium starch glycolate, starch, pregelatinised starch and combinations thereof.

6. The solid oral composition of claim 1, wherein the solid oral dosage form comprises 22-27 wt% of maraviroc, 50-75 wt% of the diluent, 1-8 wt% of the disintegrant, 0.5 wt% to 5 wt% of the colloidal silicon dioxide as a dispersing agent, optionally 0.5-2 wt% of a lubricant, and optionally 1-4 wt% of a coating material, based on the total weight of the tablet.

7. The solid oral composition according to claim 1 in the form of a tablet composition which comprises, based on the total weight of the tablet,
i) 20 wt% to 30 wt% of amorphous maraviroc having a particle size d90 not more than 150µm, the particle size D90 is 90th volume percentile as determined using laser diffraction method.;
ii) 0.5 wt% to 5 wt% of a colloidal silicon dioxide dispersing agent;
iii) 40 wt% to 80 wt% of a diluent; and
iv) 1 wt% to 8 wt% of a disintegrant,
where in the tablet is prepared either by direct compression or dry granulation by slugging.

8. The solid oral composition of claim 1, further comprising a film coating.

9. The solid oral composition of claim 1, in the form of a tablet composition comprising maraviroc microcrystalline cellulose, lactose, colloidal silicon dioxide, magnesium stearate and one more disintegrants selected from croscarmellose sodium, sodium starch glycolate, crospovidone and polacrilin potassium.

10. A process for the preparation of a tablet composition according to claim 1 comprising maraviroc, comprising: (i) dry mixing the maraviroc with a diluent, a disintegrant and colloidal silicon dioxide to form a dry mix, (ii) lubricating the blend with a lubricant, and (iii) compressing the lubricated blend of step (ii) into tablets.

11. A process for the preparation of a tablet composition according to claim 1 comprising maraviroc comprising (i) sifting and blending the maraviroc with a diluent, a disintegrant and colloidal silicon dioxide, (ii) compressing the blended mixture of step (i) to form slugs, (iii) sizing the slugs to form granules; (iv) blending the granules with at least one excipient selected from a binder, a lubricant, a dispersing agent, a disintegrant and a glidant; and finally (v) compressing the granules of step (iv) in to tablets.

12. The composition according to claim 1 and 9, wherein maraviroc is in amorphous form.

## Patentansprüche

1. Eine feste orale Zusammensetzung umfassend Maraviroc, ein Streckmittel, einen Zerfallsbeschleuniger und kolloidales Siliziumdioxid als Dispergiermittel, wobei die feste orale Zusammensetzung entweder durch Direktverpressung oder durch Trockengranulierung hergestellt wird.

2. Die feste orale Zusammensetzung nach Anspruch 1 ausgewählt aus einer Tablette, einer Kapsel und einem Granulat.

3. Die feste orale Zusammensetzung nach Anspruch 1, weiterhin umfassend ein Bindemittel, ein Schmiermittel, ein Fließregulierungsmittel oder eine Kombination davon.

4. Die feste orale Zusammensetzung nach Anspruch 1, wobei das Streckmittel ausgewählt aus der Gruppe bestehend aus Laktose, dibasischem Calciumphosphat, Saccharose, Glucose, Mannitol, Sorbitol, Calciumcarbonat, Stärke, mikrokristalliner Cellulose, Cellulosederivaten, und Kombinationen davon ist.

5. Die feste orale Zusammensetzung nach Anspruch 1, wobei der Zerfallsbeschleuniger ausgewählt ist aus Croscarmellose-Natrium, Crospovidon, Polacrilin-Kalium, Calciumsilicat, Natrium-Carboxymethylcellulose, Natriumstärkeglycolat, Stärke, vorgelatinierter Stärke und Kombinationen davon.

6. Die feste orale Zusammensetzung des Anspruchs 1, wobei die feste orale Arzneiform 22-27 Gew.-% Maraviroc, 50-75 Gew.-% des Streckmittels, 1-8 Gew.-% des Zerfallsbeschleunigers, von 0,5 Gew.-% bis 5 Gew.-% des kolloidalen Siliziumdioxids als Dispergiermittel, wahlweise 0,5-2 Gew.-% von einem Schmiermittel und wahlweise 1-4 Gew.-% von einem Beschichtungsmaterial, bezogen auf das gesamte Gewicht der Tablette, umfasst.

7. Die feste orale Zusammensetzung nach Anspruch 1 in der Form von einer Tablettenzusammensetzung, welche, bezogen auf das gesamte Gewicht der Tablette, folgendes umfasst,
i) 20 Gew.-% bis 30 Gew.-% von amorphem Maraviroc mit einer d90-Partikelgröße, die nicht höher als 150 µm beträgt, wobei die D90-Partikelgröße das mittels eines Laserbeugungsverfahrens festgestellte 90. Volumenperzentil ist;
ii) 0,5 Gew.-% bis 5 Gew.-% von einem auf kolloidalem Siliziumdioxid basierenden Dispergierm ittel;
iii) 40 Gew.-% bis 80 Gew.-% von einem Streckmittel; und
iv) 1 Gew.-% bis 8 Gew.-% von einem Zerfallsbeschleuniger,
wobei die Tablette entweder durch Direktverpressung oder durch Trockengranulierung durch Brikettierung ("Slugging") hergestellt wird.

8. Die feste orale Zusammensetzung des Anspruchs 1, weiterhin umfassend eine Filmbeschichtung.

9. Die feste orale Zusammensetzung des Anspruchs 1, in der Form von einer Tablettenzusammensetzung umfassend Maraviroc, mikrokristalline Cellulose, Laktose, kolloidales Siliziumdioxid, Magnesiumstearat und einen oder mehrere Zerfallsbeschleuniger, die aus Croscarmellose-Natrium, Natriumstärkeglycolat, Crospovidon und Polacrilin-Kalium ausgewählt sind.

10. Ein Verfahren zur Herstellung von einer Tablettenzusammensetzung nach Anspruch 1 umfassend Maraviroc, umfassend: (i) die Trockenmischung des Maravirocs mit einem Streckmittel, einem Zerfallsbeschleuniger und kolloidalem Siliziumdioxid, um eine Trockenmischung zu bilden, (ii) das Schmieren der Mischung mit einem Schmiermittel, und (iii) das Komprimieren der geschmierten Mischung des Schritts (ii), um Tabletten zu bilden.

11. Ein Verfahren zur Herstellung von einer Tablettenzusammensetzung nach Anspruch 1 umfassend Maraviroc, umfassend (i) das Sieben und das Vermischen des Maravirocs mit einem Streckmittel, einem Zerfallsbeschleuniger und kolloidalem Siliziumdioxid, (ii) das Komprimieren der vermischten Mischung des Schritts (i), um Briketten zu bilden, (iii) die Größensortierung der Briketten, um Granulate zu bilden; (iv) das Vermischen der Granulate mit mindestens einem Hilfsstoff, der aus einem Bindemittel, einem Schmiermittel, einem Dispergiermittel, einem Zerfallsbeschleuniger und einem Fließregulierungsmittel ausgewählt ist; und schließlich (v) das Komprimieren der Granulate des Schritts (iv), um Tabletten zu bilden.

12. Die Zusammensetzung nach Anspruch 1 und 9, wobei das Maraviroc in amorpher Form ist.

## Revendications

1. Une composition orale solide comprenant du maraviroc, un diluant, un agent de désagrégation, et du dioxyde de silicium colloïdal comme agent de dispersion, dans laquelle la composition orale solide est préparée ou bien moyennant compression directe ou bien moyennant granulation par voie sèche.

2. La composition orale solide selon la revendication 1, choisie parmi un comprimé, une capsule et un granulé.

3. La composition orale solide selon la revendication 1, comprenant en outre un liant, un agent lubrifiant, un agent de glissement, ou une combinaison de ceux-ci.

4. La composition orale solide selon la revendication 1, dans laquelle le diluant est choisi dans le groupe constitué du lactose, du phosphate de calcium dibasique, du saccharose, du glucose, du mannitol, du sorbitol, du carbonate de calcium, de l'amidon, de la cellulose microcristalline, des dérivés de cellulose, et des combinaisons de ceux-ci.

5. La composition orale solide selon la revendication 1, dans laquelle l'agent de désagrégation est choisi parmi le croscarmellose de sodium, la crospovidone, la polacriline de potassium, le silicate de calcium, la carboxyméthylcellulose de sodium, le glycolate d'amidon de sodium, l'amidon, l'amidon prégélatinisé et des combinaisons de ceux-ci.

6. La composition orale solide de la revendication 1, dans laquelle la forme galénique orale solide comprend 22-27 % en poids de maraviroc, 50-75 % en poids de diluant, 1-8 % en poids de l'agent de désagrégation, de 0,5 % en poids à 5 % en poids du dioxyde de silicium colloïdal comme agent de dispersion, facultativement 0,5-2 % en poids d'un agent lubrifiant et facultativement 1-4 % en poids d'un matériau de revêtement, par rapport au poids total du comprimé.

7. La composition orale solide selon la revendication 1 en forme d'une composition de comprimé qui comprend, par rapport au poids total du comprimé,
i) de 20 % en poids à 30 % en poids de maraviroc amorphe ayant une taille des particules d90 qui n'est pas supérieure à 150 µm, où la taille des particules D90 est le 90^{e} percentile en volume tel que déterminé en utilisant un procédé moyennant diffraction laser ;
ii) de 0,5 % en poids à 5 % en poids d'un agent de dispersion de dioxyde de silicium colloïdal ;
iii) de 40 % en poids à 80 % en poids d'un diluant ; et
iv) de 1 % en poids à 8 % en poids d'un agent de désagrégation,
dans laquelle le comprimé est préparé ou bien moyennant compression directe ou bien moyennant granulation par voie sèche moyennant « slugging » (briquetage).

8. La composition orale solide de la revendication 1, comprenant en outre un revêtement en film.

9. La composition orale solide de la revendication 1, en forme d'une composition de comprimé comprenant du maraviroc, de la cellulose microcristalline, du lactose, du dioxyde de silicium colloïdal, du stéarate de magnésium et un ou plusieurs agents de désagrégation choisis parmi le croscarmellose de sodium, le glycolate d'amidon de sodium, la crospovidone et la polacriline de potassium.

10. Un procédé de préparation d'une composition de comprimé selon la revendication 1 comprenant du maraviroc, comprenant : (i) mélanger à sec le maraviroc avec un diluant, un agent de désagrégation et du dioxyde de silicium colloïdal afin de former un mélange à sec, (ii) lubrifier le mélange avec un agent lubrifiant, et (iii) comprimer le mélange lubrifié de l'étape (ii) en formant des comprimés.

11. Un procédé de préparation d'une composition de comprimé selon la revendication 1 comprenant du maraviroc comprenant (i) tamiser et mêler le maraviroc avec un diluant, un agent de désagrégation et du dioxyde de silicium colloïdal, (ii) comprimer le mélange mêlé de l'étape (i) afin de former des briquettes, (iii) classer les briquettes selon la taille afin de former des granulés ; (iv) mêler les granulés avec au moins un excipient choisi parmi un liant, un agent lubrifiant, un agent de dispersion, un agent de désagrégation et un agent de glissement ; et, finalement, (v) compresser les granulés de l'étape (iv) en formant des comprimés.

12. La composition selon la revendication 1 et 9, dans laquelle le maraviroc est en forme amorphe.
